# EUROPÄISCHE PATENTANMELDUNG

(11) **EP 0 997 532 A2**
(43) Veröffentlichungstag der Anmeldung: **03.05.2000**
(21) Anmeldenummer: 99120741.6
(22) Anmeldetag: 20.10.1999
(51) Int. Cl.: C12P 13/08, C12P 13/06, C12N 1/38

(54) **Verfahren zur fermentativen Herstellung von L-Aminosäuren**

(30) Priorität: 28.10.1998 DE 19849625
(71) Anmelder: Degussa-Hüls Aktiengesellschaft, 60287 Frankfurt am Main (DE)
(72) Erfinder: Becker, Ulrich, Dr., 97611 Selce (SK); Peter, Heidi, Dr., 42799 Leichlingen (DE); Morbach, Susanne, Dr., 52428 Jülich (DE); Walger, Ilona, Dr., 33613 Bielefeld (DE); Krämer, Reinhard, Prof. Dr., 52428 Jülich (DE); Pfefferle, Walter, Dr., 33790 Halle (DE)

(57) **Zusammenfassung**

Die Erfindung betrifft ein Verfahren zur fermentativen Herstellung von L-Aminosäuren mit coryneformen Bakterien, bei dem man der Fermentationsbrühe L-Prolin als osmoprotektive Substanz zusetzt, um die Auswirkungen des hyperosmotischen Stresses auf die Zellen zu dämpfen.

## Beschreibung

Die Erfindung betrifft ein Verfahren zur fermentativen Herstellung von L-Aminosäuren mit coryneformen Bakterien, bei dem man der Fermentationsbrühe L-Prolin als osmoprotektive Substanz zusetzt.

### Stand der Technik

Es ist bekannt, daß die meisten Mikroorganismen unter osmotischem Streß Kaliumionen oder sogenannte Osmolyte (organische Verbindungen) in ihrem Cytoplasma anreichern. Dies führt zu einer internen osmotischen Widerstandskraft, die der Dehydratisierung der Zellen vorbeugt.
In diesem Zusammenhang ist bekannt, daß durch Zugabe von Glycin-Betain die Wachstumsrate der Zellen besonders in Medien mit inhibierendem osmotischem Streß stimuliert wird. Dies führt zu einer Erhöhung der Zuckerverbrauchsrate und einer Zunahme der L-Lysin Produktion (Y. Kawahara, Y. Yoshihara, S. Ikeda, H. Yoshii, Y. Hirose, Stimulatory effect of glycine betaine on L-lysine fermentation (1990), 34 (1), S. 87-90, Applied Microbiology Biotechnology)

Bei Prolin-auxotrophen Mutanten von Brevibacterium lactofermentum wurde gefunden, daß Prolin bei der Osmoregulation eine Rolle spielt.

Die osmotische Toleranz dieser Stämme erwies sich niedriger als die des Wildstammes.
In diesem Zusammenhang zeigt sich die Aktivität der Pyrrolin-5-Carboxylat Reduktase als um das Dreifache gesteigert, wenn die Zellen unter osmotischem Streß wuchsen. (Y. Kawahara, T. Ohsumi, Y. Yoshihara, S. Ikeda, Proline in the Osmoregulation of Brevibacterium lactofermentum, (1989), 53, (9), S. 2475-2479, Agricultural and Biological Chemistry).
Die Herstellung von Aminosäuren ist diesem Zitat nicht zu entnehmen.

### Aufgabe der Erfindung

Die Aufgabe der Erfindung besteht darin, ein Verfahren zur fermentativen Herstellung von L-Aminosäuren zur Verfügung zu stellen, bei dem die Auswirkungen des hyperosmotischen Stresses auf die Zellen gedämpft werden.

### Beschreibung der Erfindung

Gegenstand der Erfindung ist ein Verfahren zur fermentativen Herstellung von L-Aminosäuren, das dadurch gekennzeichnet ist, daß man L-Aminosäuren produzierende und ausscheidende coryneforme Mikroorganismen in einem Medium kultiviert, dem man neben den üblichen Bestandteilen, bevorzugt zu Beginn der Fermentation, L-Prolin zusetzt. Dies gilt insbesondere für sogenannte Minimalmedien bzw. definierte Medien, die aus mengen- und stoffmäßig identifizierten Bestandteilen zusammengesetzt sind. Aber auch bei komplex zusammengesetzten Medien, die unter anderem Hydrolysate oder Extrakte enthalten, führt der Zusatz von L-Prolin zu verbesserten Ausbeuten.

L-Prolin dient dabei nicht als C- oder N-Quellen im Stoffwechsel der Mikroorganismen. Der Zusatz bewirkt aber ein verbessertes Wachstum der Aminosäureproduzenten und eine Erhöhung der L-Aminosäure-Ausbeute.

Coryneforme Mikroorganismen, insbesondere der Art Corynebacterium glutamicum, sind als Aminosäureproduzenten seit langem bekannt. Bevorzugt werden geeignete Stämme zur Herstellung von L-Lysin, L-Isoleucin, L-Threonin oder L-Valin eingesetzt. Auch L-Glutaminsäure kann so produziert werden.

Die Fermentation führt man im allgemeinen bei Temperaturen zwischen 25 und 50 ° C, vorzugsweise 30 bis 45 ° C durch, während der pH-Wert zwischen 6 und 8, vorzugsweise 7 - 7,5 und die Ammoniumkonzentration bevorzugt zwischen 0,5 und 8 g/l liegt.

Man setzt der Fermentationsbrühe L-Prolin in einer Menge zwischen 0,01 bis 10 g/l, bevorzugt zwischen 0,1 und 2,5 g/l zu.

Geeignete Stämme der Gattung Corynebacterium, insbesondere der Art Corynebacterium glutamicum sind beispielsweise die bekannten Glutaminsäure-produzierenden Wildtypstämme
Corynebacterium glutamicum ATCC13032
Corynebacterium acetoglutamicum ATCC15806
Corynebacterium acetoacidophilum ATCC13870
Brevibacterium flavum ATCC14067
Brevibacterium lactofermentum ATCC13869 und
Brevibacterium divaricatum ATCC14020
   und daraus hergestellte Mutanten bzw. Stämme,
   wie beispielsweise die L-Lysin produzierenden Stämme
Corynebacterium glutamicum FERM-P 1709
Brevibacterium flavum FERM-P 1708 und
Brevibacterium lactofermentum FERM-P 1712
   oder wie beispielsweise die L-Threonin produzierenden Stämme
Corynebacterium glutamicum FERM-P 5835
Brevibacterium flavum FERM-P 4164 und
Brevibacterium lactofermentum FERM-P 4180
   oder wie beispielsweise die L-Isoleucin produzierenden Stämme
Corynebacterium glutamicum FERM-P 756
Brevibacterium flavum FERM-P 759 und
Brevibacterium lactofermentum FERM-P 4192
   oder wie beispielsweise die L-Valin produzierenden Stämme
Brevibacterium flavum FERM-P 512 und
Brevibacterium lactofermentum FERM-P 1845

Zur Fermentation verwendet man man bekannte Basismedien für die Produktion von L-Aminosäuren, wie sie in der vorliegenden Erfindung genannt sind, oder herkömmliche Medien für die Produktion von L-Aminosäuren, die für L-Aminosäure-produzierende Bakterien geeignet sind.

Als Hauptkohlenstoffquellen werden bekannterweise Zucker verwendet, wie Glukose, Saccharose, Fruktose, Maltose, Melassen, aber auch Stärke und Stärkehydrolysat, Cellulose und verzuckerte Cellulose, Laktose, Fettsäuren wie Essigsäure, Propionsäure, Palmitinsäure, Stearinsäure, Linolsäure; organische Säuren wie Brenztraubensäure, Citronensäure, Bernsteinsäure, Fumarsäure, Äpfelsäure; Alkohole wie Ethylalkohol, Butylalkohol; einzelne Komponenten oder Mischungen der genannten Verbindungen. Zusätzlich können Precursor aus dem Biosyntheseweg der gewählten L-Aminosäure und diese selbst verwendet werden.

Als Phosphorquelle dienen im allgemein Phosphorsäure, Kaliumdihydrogenphosphat oder Dikaliumhydrogenphosphat oder die entsprechenden Natrium-haltigen Salze.

Als Stickstoffquellen setzt man, wie allgemein bekannt, Ammoniumsalze wie Ammoniumsulfat, Ammoniumchlorid, Ammoniumnitrat, Ammoniumacetat, Harnstoff, flüssiges Ammonium oder Ammoniakwasser ein. Als komplexe organischen Stickstoffquellen kommen Casaminoacids, Maisquellwasser, Sojamehlhydrolysat, Hefeextrakt, Biomassehydrolysate sowie Proteinhydrolysate zum Einsatz.

Als anorganische Salze können Phosphate, Magnesiumsalze, Calziumsalze, Kaliumsalze, Natriumsalze, Eisensalze, Mangansalze, Zinksalze, Kupfersalze und andere Spurenelemente wenn notwendig verwendet werden. Weiter, wenn notwendig, können Vitamine wie Biotin, Thiamin, etc. eingesetzt werden.

Die Kultivierungsbedingungen gemäß der vorliegenden Erfindung sind dieselben wie in bekannten Aminosäure-Fermentationen. Während die Zusammensetzungen der Fermentationsbrühen variieren, abhängig von der L-Aminosäure oder dem verwendeten Stamm, beträgt die Kultivierungstemperatur 25 bis 50 ° C, vorzugsweise 30 bis 45 ° C. In Bezug auf den pH-Wert erzielt man gute Ergebnisse, wenn der pH-Wert im neutralen Bereich bleibt. Bei Verwendung von Proteinhydrolysat als komplexer Stickstoffquelle wird vorteilhaft der gegebenenfalls darin enthaltene Prolingehalt bei der Bemessung des zusätzlich eingesetzten Prolins berücksichtigt. Die Menge an Prolin, die aus dem Hydrolysat stammt, ist durch die natürliche Zusammensetzung dieser Produkte begrenzt, so daß der Zusptz weiterer Prolinmengen im Rahmen des erfindungsgemäßen Verfahrens sich als vorteilhaft erweist.

### Beispiele

Die vorliegende Erfindung wird im folgenden anhand von Ausführungsbeispielen näher erläutert.
Zu diesem Zweck wurden Versuche mit Aminosäuren produzierenden Stämmen durchgeführt, in denen die Überlegenheit des beanspruchten Verfahrens demonstriert wird:
a) der L-Lysin produzierende Stamm Corynebacterium glutamicum DSM5715, (EP-B- 0435 132) und
b) der L-Threonin und L-Isoleucin produzierende Stamm Brevibacterium flavum DSM5399 (EP-B- 0385 940)

### Beispiel 1

### Fermentative Herstellung von L-Lysin

Ein Anzuchtmedium, welches 2,5 g/l NaCl, 10 g/l Pepton und 10 g/l Hefeextrakt enthält, wurde auf pH 7,4 mit Natriumhydroxid eingestellt und nach Hitzesterilisation mit 40 ml 50%-iger Glukoselösung pro Liter versetzt. 47 ml-Portionen des Mediums wurden mit einem Ösenabstrich einer 48 Stunden inkubierten Agar-Platte mit Hirn-Herzagar als Nährboden mit Corynebacterium glutamicum DSM5715 inokuliert und 20 Stunden bei 33 ° C auf einem RC-1-TK Inkubator der Firma Infors AG (Bottmingen, Schweiz)mit 150 rpm geschüttelt. Anschließend wurden die Zellen mit steriler physiologischer Kochsalzlösung gewaschen. Die Separation der Zellen erfolgte bei 4000 rpm in einer Beckmann Zentrifuge J 6B für 20 min.

Für die Hauptschüttelkolbenkultivierung wurden in einem 1 l Becherglas 40 g (NH₄)₂SO₄, 0,5 g KH₂PO₄, 0,5 g K₂HPO₄, 0,25 g MgSO₄x7 H₂O und 0,3 g L-Leucin abgewogen und 750 ml destilliertes H₂O dazugegeben. Außerdem wurden 1 ml einer Spurensalzlösung zugefügt. Die Spurensalzlösung enthielt 1,0 g FeSO₄x7H₂O, 1,0 g MnSO₄xH₂O, 0,1 g ZnSO₄x7H₂O, 0,02 g CuSO₄ und 0,002 g NiCl₂x6H₂O, welche in 100 ml dest. H₂O, zur besseren Löslichkeit der Salze mit einigen Tropfen HCl leicht angesäuert, gelöst wurden. Zusätzlich wurde 1 ml einer 0,02 g pro 100 ml dest. H₂O Biotinlösung zugegeben. Anschließend wurde NaCl in einer Konzentration von 5 g/l zugegeben. Dieses Kultivierungsmedium wurde in 45 ml Portionen in 500 ml Erlenmeyerkolben verteilt und auf unterschiedliche Konzentrationen an Prolin von 0,1 bis 10 g/l eingestellt. Nach einer Hitzesterilisation im Autoklaven bei 121°C für 20 Minuten wurden in jeden Kolben 12 ml einer separat sterilisierten 50%igen Glukoselösung und 1,2 g sterilisiertes CaCO₃ dazu gegeben. Anschließend wurde mit den, unter sterilen Bedingungen gewaschenen Zellen des Anzuchtmediums beimpft. Die optische Dichte (Messwellenlänge: 535 nm) der gewaschenen Zellen betrug 18,5; 7,7 ml dieser Suspension wurden zum Beimpfen der 57 ml Kulturmedium verwendet.

Die Kultivierung erfolgte für 72 Stunden bei 33°C und 150 rpm auf einem RC-1-TK Inkubator der Firma Infors AG (Bottmingen, Schweiz). Im Anschluß daran wurden die optische Dichte (OD) (Photometer LP2W der Firma Dr. Lange; Berlin, Deutschland) und die Konzentration an gebildeter L-Aminosäure in der Kultursuspension bestimmt. Aminosäuren wurden mit einem Aminosäureanalysator der Firma Eppendorf-BioTronik (Hamburg, Deutschland) durch Ionenaustauschchromatographie und Nachsäulenreaktion mit Ninhydrinandetektion bestimmt. In Tabelle 1 ist das Ergebnis des Versuchs dargestellt.

**TABELLE 1**

| Prolin [g/l] | OD 535 nm | Lysin [g/l] |
|---|---|---|
| 0 | 24,6 | 23,6 |
| 0,5 | 30,5 | 29,4 |

### Beispiel 2

### Fermentative Herstellung von L-Threonin

Ein Kulturmedium, welches 100 g/l Saccharose, 12 g/l (NH₄)₂SO₄, 100 ml/l Sojabohnenmehlhydrolysat, 0,5 g/l K₂HPO₄, 0,5 g/l KH₂PO₄, 0,25 g/l MgSO₄x7H₂O, 5,0 g/l NaCl und 1 ml Spurensalzlösung enthält wurde auf pH 7,0 engestellt und autoklaviert. Die Spurensalzlösung bestand aus 1,0 g FeSO₄ x 7 H₂O, 1,0 g MnSO₄ x H₂O, 0,1 g ZnSO₄ x 7 H₂O, 0,02 g CuSO₄, 0,002 g NiCl₂ x 6 H₂O welche auf 100 ml mit demineralisiertem Wasser und einigen Tropfen einer 1 N HCl-Lösung aufgefüllt wurde.

Zu dem Kulturmedium wurden jeweils 1 ml einer Biotin- bzw. Thiamin-Stammlösung mit 0,2 mg/l sterilfiltriert zugegeben. 10,0 g/l CaCO₃ wurde mit den Schüttelkolben gemeinsam sterilisiert. Die Prolin-Konzentration des Kulturmediums, die aus dem Einsatz des Sojabohnenmehlhydrolysates resultierte, betrug 0,34 g/l. Aus einer Prolin-Stammlösung wurde die angegebene Konzentration an Prolin sterilfiltriert in das Medium zugegeben.

Eine mit DSM5399 für 72 Stunden inkubierte Agarplatte mit Hirn-Herzagar als Nährboden wurde mit 10 ml steriler physiologischer Kochsalzlösung aufgeschwemmt. 100 ml Erlenmeyer-Schüttelkolben wurden mit 10 ml Kultivierungsmedium befüllt und mit 100 µl der abgeschwemmten Zellsuspension inokuliert.
Die Kultivierung erfolgte für 72 Stunden bei 30°C und 300 rpm. Im Anschluß daran wurden die OD bei einer Messwellenlänge von 660 nm und die Threoninkonzentration, wie in Beispiel 1 angegeben, gemessen. In Tabelle 2 ist das Ergebnis des Versuchs dargestellt.

**TABELLE 2**

| Prolin [g/l] | OD 660 nm | Threonin [g/l] |
|---|---|---|
| 0,34 | 51,2 | 0,63 |
| 0,66 | 52,6 | 1,29 |

### Beispiel 3

### Fermentative Herstellung von L-Isoleucin

Ein Kulturmedium, welches 100 g/l Saccharose, 12 g/l (NH₄)₂SO₄, 0,5 g/l K₂HPO₄, 0,5 g/l KH₂PO₄, 0,25 g/l MgSO₄x7H₂O, 5,0 g/l NaCl und 1 ml Spurensalzlösung enthält wurde auf pH 7,0 engestellt und autoklaviert. Die Spurensalzlösung bestand aus 1,0 g FeSO₄ x 7 H₂O, 1,0 g MnSO₄ x H₂O, 0,1 g ZnSO₄ x 7 H₂O, 0,02 g CuSO₄, 0,002 g NiCl₂ x 6 H₂O welche auf 100 ml mit demineralisiertem Wasser und einigen Tropfen einer 1 N HCl-Lösung aufgefüllt wurde.

Zu dem Anzuchtmedium wurden jeweils 1 ml einer Biotin- bzw. Thiamin-Stammlösung mit 0,2 mg/l sterilfiltriert zugegeben. 10,0 g/l CaCO₃ wurde mit den Schüttelkolben gemeinsam sterilisiert. Aus einer Prolin-Stammlösung wurde die entsprechende Konzentration sterilfiltriert in das Kulturmedium zugegeben.

Eine mit DSM5399 für 72 Stunden inkubierte Agarplatte mit Hirn-Herzagar als Nährboden wurde mit 10 ml steriler physiologischer Kochsalzlösung aufgeschwemmt. 100 ml Erlenmeyer-Schüttelkolben wurden mit 10 ml Kultivierungsmedium befüllt und mit 100 µl der abgeschwemmten Zellsuspension inokuliert.
Die Kultivierung erfolgte für 72 Stunden bei 30°C und 300 rpm. Im Anschluß daran wurden die OD bei einer Messwellenlänge von 660 nm und die Isoleucinkonzentration, wie in Beispiel 1 angegegeben, gemessen. In Tabelle 3 ist das Ergebnis des Versuchs dargestellt.

**TABELLE 3**

| Prolin [g/l] | OD 660 nm | L-Isoleucin [g/l] |
|---|---|---|
| 0 | 51,2 | 0,18 |
| 0,1 | 52,0 | 0,36 |

## Patentansprüche

1. Verfahren zur fermentativen Herstellung von L-Aminosäuren durch Kultivieren von diesen Aminosäuren produzierenden und ausscheidenden coryneformen Mikroorganismen,
**dadurch gekennzeichnet,**
daß man der die bekannten C- und N-Quellen enthaltenden Fermentationsbrühe, bevorzugt zu Beginn der Fermentation, L-Prolin zusetzt.

2. Verfahren gemäß Anspruch 1,
**dadurch gekennzeichnet,**
daß man L-Prolin in einer Menge von 0,01 bis 10 g/l, bezogen auf die Fermentationsbrühe, zusetzt.

3. Verfahren gemäß Anspruch 2,
**dadurch gekennzeichnet,**
daß man L-Prolin in einer Menge von 0,1 bis 2,5 g/l, bezogen auf die Fermentationsbrühe, zusetzt.

4. Verfahren gemäß den Ansprüchen 1 bis 3,
**dadurch gekennzeichnet,**
daß man die Fermentation in einem Minimalmedium und/oder definiertem Medium durchführt.

5. Verfahren gemäß den Ansprüchen 1 bis 3,
**dadurch gekennzeichnet,**
daß man die Fermentation in einem Hydrolysat enthaltenden Medium durchführt.

6. Verfahren gemäß den Ansrüchen 1 bis 5,
**dadurch gekennzeichnet,**
daß man L-Lysin, L-Isoleucin, L-Threonin oder L-Valin herstellt.

7. Verfahren gemäß den Ansprüchen 1 bis 6,
**dadurch gekennzeichnet,**
daß man Mikroorganismen der Gattung Corynebacterium einsetzt.
